# EUROPEAN PATENT APPLICATION

(11) **EP 3 409 246 A1**
(43) Date of publication of application: **05.12.2018**
(21) Application number: 16886831.3
(22) Date of filing: 27.01.2016
(51) Int. Cl.: A61F 5/56, A61C 7/08, A61C 9/00

(54) **VIBRATORY INTRA-ORAL BIOFEEDBACK APPARATUS**

(71) Applicant: Bolzan, Marcelo Costa, 04602-060 São Paulo (BR)
(72) Inventor: Bolzan, Marcelo Costa, 04602-060 São Paulo (BR)
(74) Representative: Werner & ten Brink
(86) International application number: PCT/BR2016/050012
(87) International publication number: WO 2017/127904

(57) **Abstract**

A vibratory intra-oral biofeedback apparatus for the treatment of orofacial pain and temporo-mandibular dysfunction by providing a stimulus in response to involuntary or parafunctional contraction of the chewing muscles above a pre-set limit, comprising at least one active device formed by a set of functional elements, the latter comprising a vibrator (18), at least one sensor (15), batteries (17), and processor. Said device is sealingly inserted in a plate made of synthetic resin which in the preferred embodiments is configured according to the contour of the dental arches in which it fits. The stimulation in the form of vibration is triggered when the force of involuntary contraction of the chewing muscles reaches a threshold which generally ranges from 20 N to 70 N. The apparatus may comprise two active devices located on the left and right side of the arch or just an active device, located on one side, left or right, of the arch.

## Description

### Field of the invention

The present invention refers to the field of dentistry, more specifically to the treatment of orofacial pain and temporomandibular disorders, regarding intraoral devices which provide a stimulus in response to involuntary or parafunctional contraction of the masticatory muscles above a preset limit of contraction force.

### Background of the invention

The problems of orofacial pain, tension headaches, temporomandibular disorders and exacerbated bruxism, which the invention intends to treat or control, can be treated by several proposed techniques, with varying degrees of success.

The following list comprises the most common treatment methods:
- Non-opioid analgesics;
- Opioid analgesics;
- Muscle relaxants;
- Benzodiazepines;
- Occlusal splints;
- Physiotherapies;
- Extracorporeal biofeedback devices;
- Intraoral biofeedback devices;
- Laser;
- Botulinic toxin;
- Anti-inflammatory drugs;
- Psychotherapy;
- Surgeries.

Drugs in general have good immediate effect and low cost. In some cases, however, they may have unbearable side effects, bringing about the need for combination with other drugs. Other drugs may produce long-term dependency, or even some localized physical problems.

Occlusal splints, because they are made in laboratories and require a lot of time of skilled labor, have a high cost, limited range of action and may produce undesirable anatomical changes in the long term. In addition, they present, in common with physiotherapeutic and psychotherapeutic treatments, the need for frequent visits to the therapist.

Physiotherapy and psychotherapy are considered more as adjuncts to other treatments, and are effective only if associated with other treatment methods and have a transient effect.

*Botulinum* toxins, although effective in very specific cases, have a high cost and limited duration (4 to 6 months).

Surgery is usually the final option, when other treatments have not been effective, but it is expensive and very invasive.

Therefore, extra-oral and intraoral biofeedback devices appear to be the most adequate option. In fact, they are relatively non-invasive and relatively inexpensive - because they are industrially produced - easy to apply, require short installation and adaptation times and do not require readjustment sessions, have no known side effects, do not cause long-term problems, and have a fairly large efficacy verified in hundreds of tests as well as scientific papers published in international journals.

Among the prior art documents describing such devices, the following may be cited.

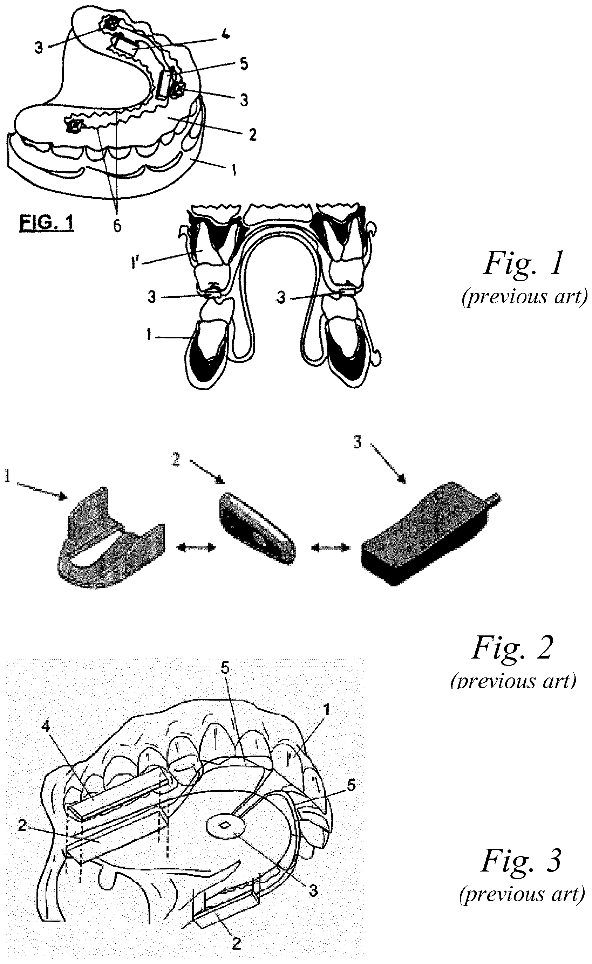

ES1060938 (U) "*Férula Electrónica* para *Pacientes Bruxistas y*/*o Apretadores*" describes a device, shown in Fig. 1, mounted on a base or support (2) on which the sensor elements (3) are in direct contact with the teeth and detect pressures above a certain value, a vibratory element (5) being optionally provided with means for transmitting RF signals to an external receiver. Among the drawbacks of this device is the fact that the sensors are in direct contact with the teeth, which can impair their physical integrity, as well as the fact that the vibrator (5) is located inside the upper dental arch. Moreover, its functioning depends on the communication with external processors that will record and analyze bruxism and then transmit the stimulus signal in a way not specifically described in this document. Further, the description and the illustration included therein indicate that the apparatus is made of acrylic resin from a previously cast gypsum model.

WO2009013371 "*Sistema* para *la Detección y el Tratamiento del Bruxismo y Otras Patologías Oclusales*" describes a system, shown in Fig. 2, comprising an intraoral support (1) which detects bruxism and transmits the corresponding information to an external controller (3) that analyzes the information and produces a stimulus such as an acoustic, vibrating or luminous signal. A drawback of the system lies in the fact that its operation depends on the bidirectional communication between the intraoral plate (1) and the external controller (3). In addition, the document does not detail the positioning of the sensors, an additional drawback being that the splint is integral and made of polymeric material (composite resin). Furthermore, the subject matter is limited to the detection of bruxism through sensors embedded in the acrylic splint, not providing treatment for orofacial pain and headaches.

WO9830769 "*Dispositivo Electronico de Transmisión de Datos para Férulas de Presión en Pacientes Bruxistas*" discloses a device mounted on a splint adapted to one of the dental arches. In the embodiment shown in Fig. 3, the splint is adapted to the upper dental arch 1 and comprises sensors (2) enclosed between waterproof plastic sheets (4), a transmitter (3) which receives pressure-related data through the wires (5). Said transmitter communicates through an RF link with a receiver (not shown), which triggers stimulus means (not described in the document). In addition to the shortcomings due to the need for an RF link, due to the use of an external receiver, this publication does not detail the means used to produce the stimulus.

### Objects of the invention

In view of the shortcomings and disadvantages of the prior art, it is a first object of the invention to provide an apparatus for treating orofacial pain and temporomandibular disorders by applying intraoral stimulation when the contraction force of the masticatory muscles exceeds a preset level, whether static or dynamic.

Another object is to provide an intraoral oral biofeedback device that allows treating patients with tension headaches, migraine, orofacial pain, temporo-mandibular dysfunctions and bruxism, either static or dynamic.

### Summary of the invention

The above mentioned objects are attained by the invention by means of a system comprising a device that produces intraoral vibrations in response to the detection, by sensing means, of a biting force above a specified threshold.

According to another feature of the invention, said sensing means detects vertical as well as horizontal movements of the jaw in any direction.

According to yet another feature of the invention, the device herein proposed is manufactured in several sizes and can be fitted into the mouth by the dentist or the patient himself. In addition, the device is an autonomous unit, i.e., all its elements (sensors, batteries, vibrator and processor) are included in the unit.

### Description of the figures

Further advantages and features of the invention will become more apparent from the description of preferred exemplary embodiments and corresponding figures, given as non-limiting examples, wherein:
Figures 1, 2 and 3 show previous art devices for treating bruxism.
Figures 4-a and 4-b show a first embodiment of the device, according to the principles of the present invention.
Figures 5-a, 5-b and 5-c show a second embodiment of the device, according to the principles of the present invention.
Figures 6-a, 6-b and 6-c show a third embodiment of the device, according to the principles of the present invention.
Figure 7 shows a detailed view of the device placement on the dental arch.
Figure 8 shows a fourth embodiment of the device, according to the principles of the present invention.

### Detailed description of the invention

By filling in the gaps of the existing options, the poposed device, which is based on an innovative principle, elicits a quite favorable reaction on the part of the patient.

As mentioned, nocturnal involuntary muscle contraction is usually responsible for morning headaches, migraine aggravation, TMJ overload and lesions, facial pain, tooth sensitivity, and finally, overload off the masticatory system comprising muscles, joints, nerves and blood vessels.

Firstly, the device of the invention reduces the strength of muscle contraction, also reducing joint compression and arterial and venous overload in the region. Such a reduction has an analgesic and myo-relaxing effect and even an anti-inflammatory effect from the mechanical point of view. After the symptoms subside, the constant or eventual use of the device can prevent the return of symptoms, being an effective substitute for physiotherapy and even psychotherapy, as it teaches the patient good postural mouth customs. By preventing the problem from getting worse, it can avoid the need for future surgery.

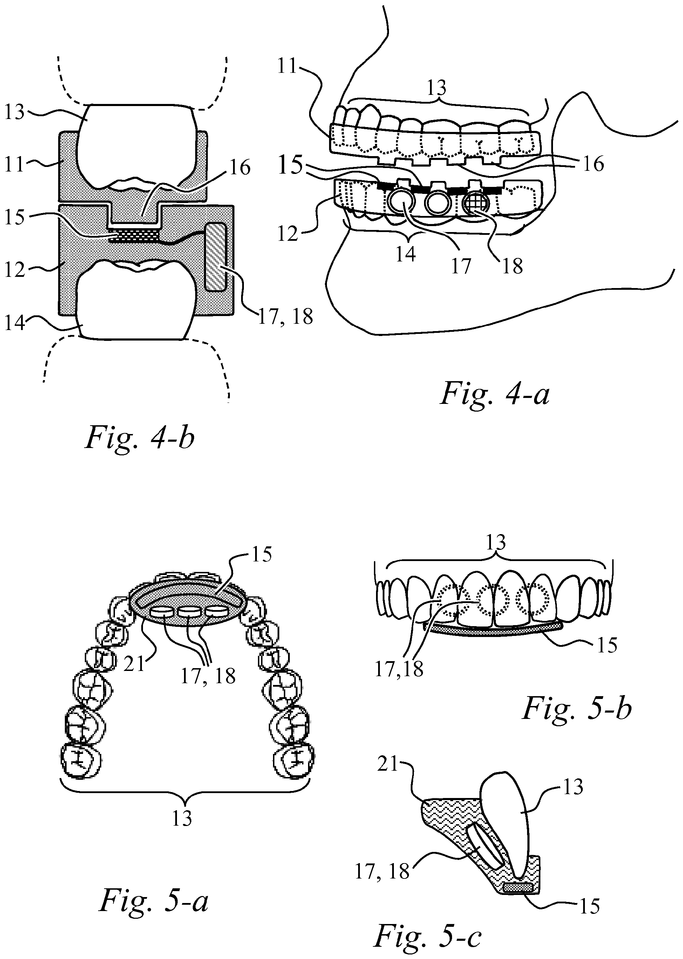

According to Figures 4-a and 4-b, the apparatus comprises an upper mouth guard 11 and a lower mouth guard 12, made of synthetic resin, respectively fitted in the upper and lower dental arches 13 and 14. In the embodiment shown, the lower mouth guard is provided with an active device comprising the sensors 15, which may comprise mechanical switches, strain gages, load cells, conductive rubber or piezoelectric sensors. Said active device also comprises a microprocessor (not shown), batteries 17 and at least one vibrating element 18 of a known type, connected to each other and to said sensors.

The upper mouth guard 11 is passive and comprises a set of protrusions 16, positioned coincidentally and in opposition to said sensors 15, so that, upon occlusion, as exemplified in Fig. 4-b, said protrusions press the sensors which produce an electrical signal whose intensity is proportional to the biting force. Said signal is analyzed by the microprocessor; if such magnitude indicates a biting force above a preset threshold, a vibrator 18 is triggered. This embodiment of the invention employs the vibratory action to treat snoring and apnea associated with protrusion of the mandible, in order to result in muscle relaxation at the same time.

These vibrations or buzzing are perceived consciously or unconsciously by the patient, causing the reflex arc mechanism to interrupt the muscle contraction. Vibration will be triggered every time the biting force exceeds the preset limit, inducing a reduction of muscle contraction during the period in which the apparatus is used. The threshold value of the biting force between the opposing teeth which results in actuation of the apparatus can generally be set between 20N and 70N, with 40N being the default value. Such a threshold can be pre-programmed during manufacturing, depending on the characteristics of the user, evaluated by the health professional. In another embodiment of the invention, the adjustment may be performed in the dentist's office.

The material used in the manufacturing of the mouth guards is preferably a synthetic resin comprising plastics of various types (polyurethanes, polycarboxylates, PET, silicone, and so on) which, in addition to providing electrical insulation between the elements, provides the watertight sealing of the assembly, a feature that is necessary in view of the conditions found in the oral cavity.

Of course, the functions of the said mouth guards can be reversed, with the active device, containing the electronic components, installed in the upper component, and the passive elements, i.e. the pressing projections 16 being provided in the lower component. Said reversal will not change the operating principles of the apparatus.

According to the invention, the apparatus may be unilateral (i.e. associated with only one side of the mouth) or bilateral, upper or lower, anterior or posterior, covering the whole or only part the dental arches. Moreover, the elements of the apparatus may be integrated in a single piece, or comprising separate parts joined by a fixed or adjustable isthmus, covering partially any region of the dental arches, from a single tooth up to all teeth.

Figures 5-a, 5-b and 5-c show an embodiment of the invention in which the sensors, instead of being located on the sides of the dental arch as exemplified in Figure 4-a, are embedded in a unit 21 placed in the front portion of the same, that is, associated with the incisor teeth. This embodiment uses only one active device placed next to the inner region of the teeth 13, in which the sensor 15, the microprocessor (not shown) and the electronic elements 17, 18 are located. The operating principle is the same as previously described, the sensor 15 being pressed by the upper edges of the lower incisors.

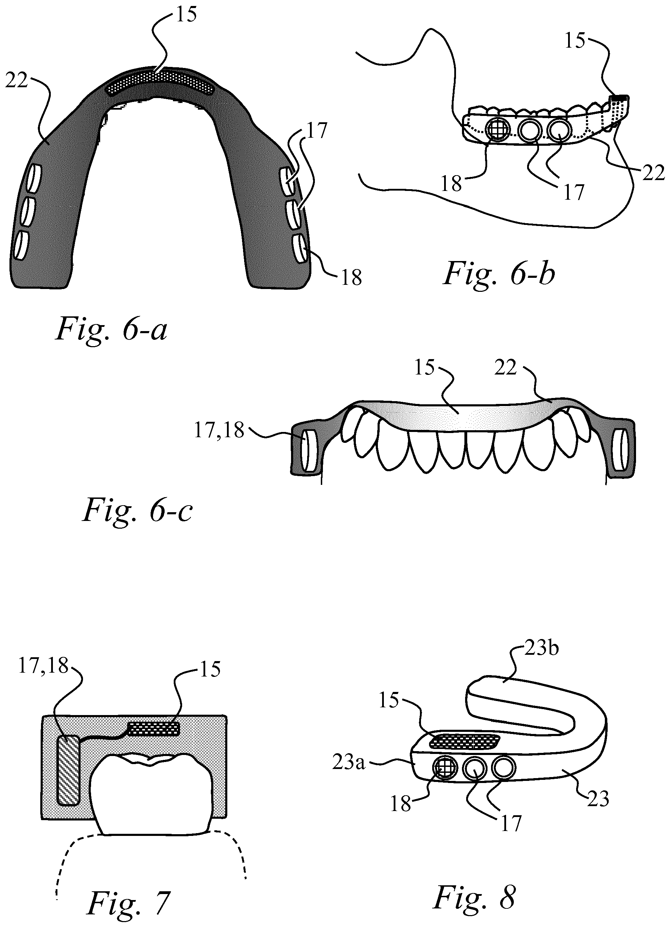

Another embodiment of the invention is shown in Figures 6-a, 6-b and 6-c. According to these figures, this embodiment employs only the lower mouth guard 22, which is installed in the lower dental arch of the patient. The sensor element 15, which covers the upper edges of the lower dental arch incisors, is electrically connected to the electronic components 17, 18, which operate in the manner described above in connection with the embodiments of Figures 4 and 5.

Although Fig. 6-a shows two active devices, located on the right and left sides of the dental arch, the apparatus may employ only one of these active devices, positioned either on the left or on the right side. Such an embodiment is shown in Fig. 8, employing only one active device situated, in this example, on the right-hand side 23a of the mouth guard 23. As shown in this figure, the mouth guard comprises a left-side branch extension 23b in order to provide a balanced occlusion.

The invention provides two ways of installing the apparatus into the patient's mouth. In a first embodiment, shown in Fig. 7, the engagement of one or both of the mouth guards is done by the dentist, using heavy or light silicone resins of addition or condensation such as acryline resin, resilient resins, or other materials available for the relining of prostheses.

In the case of installation by the patient, thermo-activated plastics will be used and, after preheating, can be molded in the mouth as is done with sports mouth guards such as, for example, those used in boxing. In such an embodiment of the invention, wherein the patient himself makes the installation, the active device is embedded in a thermo-activated synthetic resin, which is a material that softens when immersed in hot water. Once softened, the user can manipulate it in order to adapt it to his dental arch, and the apparatus will remain fixed after cooling.

While the present invention has been described in connection with preferred embodiments, it is to be understood that the invention is not to be limited to those particular embodiments. Rather, it is intended to cover all possible alternatives, modifications and equivalents within the spirit and scope of the invention.

## Claims

1. **A VIBRATORY INTRA-ORAL BIOFEEDBACK APPARATUS** for treating orofacial pain and temporomandibular disorders by providing a stimulus in response to the involuntary or parafunctional contraction of the masticatory muscles above a preset limit, **characterized in that** it comprises an autonomous unit containing at least an active device which comprises a set of functional elements comprising at least one vibrator (18), at least one sensing element (15), batteries (17) and a processor; said set of functional elements being sealingly enclosed in a synthetic resin mouth guard.

2. **AN APPARATUS** as claimed in claim 1, **characterized in that** it comprises two mouth guards (11, 12) respectively fitted in the upper (13) and lower (14) dental arches, one of which contains, sealingly inserted therein, at least such an active device.

3. **AN APPARATUS** as claimed in claim 2, **characterized in that** said lower mouth guard (12) includes said active device which comprises at least one vibrator (18) at least one sensor (15), batteries (17) and a processor, said at least one sensor being selected from the group comprising mechanical switches, strain gages, load cells, conductive rubber or piezoelectric sensors.

4. **AN APPARATUS** as claimed in claim 2, **characterized in that** said upper mouth guard (11) includes said active device which comprises at least one vibrator (18) at least one sensor (15), batteries (17) and a processor, said at least one sensor being selected from the group comprising mechanical switches, strain gages, load cells, conductive rubber or piezoelectric sensors.

5. **AN APPARATUS** as claimed in claim 3, **characterized in that** said upper mouth guard (11) comprises at least one protrusion (16), positioned coincidentally and in opposition to said at least one sensor (15).

6. **AN APPARATUS** as claimed in claim 4, **characterized in that** said lower mouth guard (12) comprises at least one protrusion, positioned coincidentally and in opposition to said at least one sensor.

7. **AN APPARATUS** as claimed in any one of claims 1 or 2, **characterized in that** it comprises two active devices located, one on the left side and one on the right side of the dental arch.

8. **AN APPARATUS** as claimed in any one of claims 1 or 2, **characterized in that** it comprises a single active device located either on the left or on the right side of the dental arch.

9. **AN APPARATUS** as claimed in claim 1, **characterized in that** it comprises a mouth guard (21) coincident with the frontal portion of the dental arch, an active device being embedded in said mouth guard.

10. **AN APPARATUS** as claimed in claim 1, **characterized in that** the stimulus provided in response to the involuntary contraction of the masticatory muscles is triggered when the occlusion force between the jaws reaches a threshold comprised between 20N and 70N.
